# EUROPEAN PATENT SPECIFICATION

(11) **EP 1 988 398 B1**
(45) Date of publication and mention of the grant of the patent: **16.12.2009**
(21) Application number: 07075322.3
(22) Date of filing: 02.05.2007
(51) Int. Cl.: G01N 33/94, C07C 217/74

(54) **Immunoassay for tramadol and its major metabolites**
Immuntest für Tramadol und seine hauptsächlichen Metaboliten
Dosage immunologique pour tramadol et ses métabolites les plus importants

(43) Date of publication of application: 05.11.2008
(73) Proprietor: Randox Laboratories Ltd., Crumlin, Co. Antrim BT29 4QY (GB)
(72) Inventor: Benchikh, Elouard, Crumlin County Antrim BT29 4QY (GB); Fitzgerald, Stephen Peter, Crumlin County Antrim BT29 4QY (GB); McConnell, Robert Ivan, Crumlin County Antrim BT29 4QY (GB); Lowry, Andrew Philip, Crumlin County Antrim BT29 4QY (GB)

(56) References cited:
- MOORE CHRISTINE ET AL: "Determination of meperidine, tramadol and oxycodone in human oral fluid using solid phase extraction and gas chromatography-mass spectrometry" J. CHROMATOGR. B ANAL. TECHNOL. BIOMED. LIFE SCI.; JOURNAL OF CHROMATOGRAPHY B: ANALYTICAL TECHNOLOGIES IN THE BIOMEDICAL AND LIFE SCIENCES MAY 1 2007, vol. 850, no. 1-2, 1 May 2007 (2007-05-01), pages 370-375, XP002442579

## Description

### FIELD OF THE INVENTION

The invention relates to an immunoassay method and kit for the detection and / or determination of tramadol, O-demethyltramadol and N-demethyltramadol in a sample. The invention also relates to novel immunogens, antibodies and conjugates, for use in said immunoassay method and kit for the detection and / or determination of tramadol, O-demethyltramadol and N-demethyltramadol in a sample. "Detection" means the qualitative analysis of the presence or absence. "Determination" means quantitative analysis of the amount.

### BACKGROUND TO THE INVENTION

Tramadol, systematic name (±)-*cis*-2-[(dimethylamino)methyl]-1-(3-methoxyphenyl)cyclohexanol hydrochloride, is a centrally acting analgesic for the treatment of moderate to severe pain. It has been shown to have a relatively weak affinity for the µ-opioid receptor compared to morphine, and its potential for abuse is considered low. Consequently, there is unrestricted access to the drug throughout Europe and it is unscheduled in the USA. However, individual reports and studies have shown that tramadol can provoke addictive and abusive behaviour (1, 2). This could be in part due to the ready accessibility of the drug owing to its unclassified status. After oral administration of a single 100 mg dose, the plasma concentration of tramadol peaks at approximately 600 ng/ml 2-3 hours later. Urinary excretion of the dose is as the unchanged drug (30%) and metabolites (60%). The major metabolites are (±)-*cis*-2-[(methylamino)methyl]-1-(3-methoxyphenyl)cyclohexanol (N-demethyltramadol) and (±)-*cis*-2-[(dimethylamino)methyl]-1-(3-hydroxyphenyl)cyclohexanol (O-demethyltramadol). Demethylation is catalysed in the liver by isoforms of cytochrome P-450. N-demethylation of tramadol is catalysed by CYP2B6 and CYP3A4, while O-demethylation is catalysed by CYP2D6 (3). The P-450 enzyme is subject to genetic polymorphism, and approximately 7% of Caucasians lack CYP2D6 and are considered to be poor metabolizers (3, 4) producing concentration levels of O-demethyltramadol below the limit of detection (<5 ng/ml) of a standard gas-chromatographic analytical assay (4). The structures of tramadol and its major metabolites are shown below in a representative *cis* configuration.

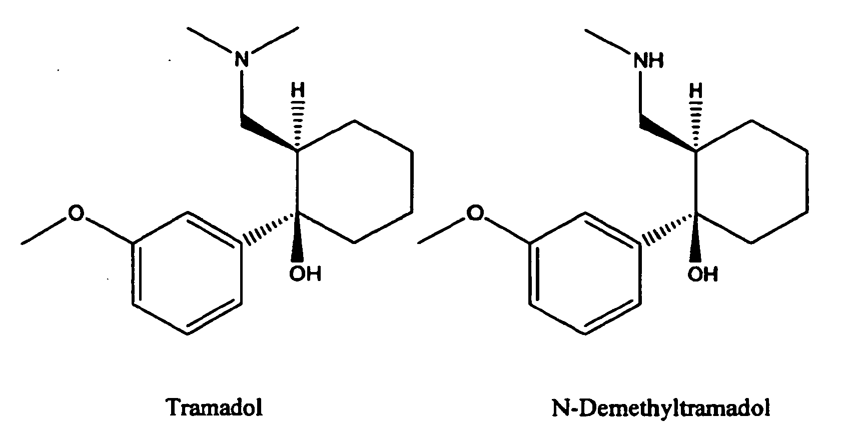

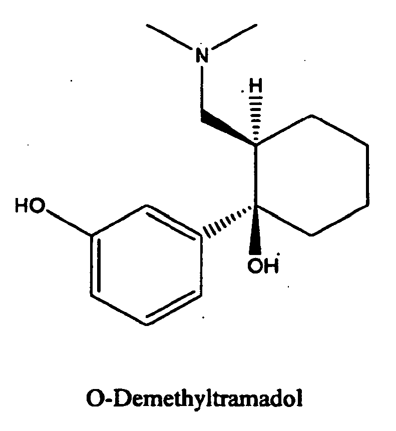

Several analytical techniques, requiring expensive equipment and highly trained staff, have been used to quantify tramadol and its metabolites in various sample types. Tao et al (5) used gas chromatography with nitrogen-phosphorous detection (GC-NPD), Leis et al (6) used gas chromatography linked to mass spectrometry (GC-MS), Zhao et al (7) used liquid chromatography with tandem mass spectrometry (LC-MS-MS), while Gu and Fawcett (8) are one of many groups who have applied high performance liquid chromatography (HPLC) in tramadol detection. In all cases at least one sample extraction step is required prior to analysis, and assay sensitivities range from 0.5 - 5 ng/ml. An assay requiring no pre-treatment step and reporting a sensitivity of 0.2 ng/ml is described by Sha et al (9). However, they utilise a specialised and expensive technique that requires a combination of headspace solid-phase microextraction (HS-SPME) with GC-MS.

Specific binding reactions, such as antibody-antigen interactions, have been used extensively in immunoassays to detect a variety of substances present in biological samples. Compared to methods such as HPLC and GC-MS, such methods are less costly and require non-specialist staff for implementation. Thus, for example, radioimmunoassays (RIAs) could be used for the determination of tramadol and its metabolites. Radioimmunoassays are very sensitive, but do require radionuclide tracers, for example ¹²⁵I and ³H. There are no known RIAs for tramadol and its metabolites. Enzyme-linked immunosorbent assays (ELISAs) are a nonradioactive alternative that could be used for the qualitative and quantitative determination of tramadol and its metabolites. Recently, four test kits for tramadol have become commercially available. Three of the kits, which detect O-demethyltramadol and tramadol, are more than 10 times less sensitive than alternative analytical methods described previously, and none of the kits are sensitive to the major metabolite N-demethyltramadol (Table 1). A further kit, produced by Immunalysis, has no available data. As previously mentioned, individuals lacking the CYP2D6 enzyme are poor at demethylating the O-methyl group of tramadol, and consequently produce levels of O-demethyltramadol of less than 5 ng/ml (4). The commercially available tramadol ELISA kits with explicit data either detect or have a high cross-reactivity towards O-demethyltramadol, and will therefore be dependent on detecting only tramadol in samples from CYP2D6 deficient individuals, which would further compromise the sensitivity of the assay.

Moore et al (12) describes the use of the Tramadol Direct Elisa Kit from Immunalysis.

It is reported that the concentration of N-demethyltramadol generally exceeds that of O-demethyltramadol. For example, N-demethyltramadol is 1.5-3 times more abundant than the O-demethyltramadol at normal tramadol dosage levels (3). Thus, as the concentration of tramadol increases (i.e. for multiple tramadol doses), the percentage ratio of the N-demethyl metabolite increases relative to the O-demethyl metabolite. An assay that also detects the N-demethyl metabolite of tramadol should increase the sensitivity of the assay, especially at lower concentrations, a characteristic especially significant in individuals lacking CYP2D6 and under-producing the O-demethyl metabolite.

Hair analysis is an alternative, non-invasive method to detect drugs of abuse, but the lower concentration levels of analytes found in hair, requires detection methods with high sensitivity. Studies to detect tramadol in hair samples require detections levels of 2 - 4 ng, for a typical 1 mg sample of hair (10, 11). Current immunoassay methods are not sufficiently sensitive to detect tramadol in hair samples.

**Table 1 Specificity and sensitivity of three commercially available ELISA kits for tramadol and its primary metabolites (The Sensitivity value applies to the most cross-reactive compound)**

| | **% Cross Reactivity** | | | **Sensitivity (ng/ml)** |
|---|---|---|---|---|
| | Tramadol | O-demethyltramadol | N-demethyltramadol | |
| IDS | 100 | 40 | <2 | 200 |
| Bio-Quant | 13 | 100 | <5 | 75 |
| Neogen | 100 | 27 | 6 | 5 |

What is required, and which is described in this invention, is an immunoassay, which detects and determines tramadol, O-demethyltramadol and N-demethyltramadol, enabling the detection of tramadol and N-demethyltramadol in CYP2D6 deficient individuals, thus ensuring maximum sensitivity. What is also required, and which is described in the invention, is an immunoassay which is as sensitive as non-immunoassay-based analytical detection methods to tramadol, O-demethyltramadol and N-demethyltramadol, allowing the detection of said analytes in biological samples at levels of less than 5 ng, as found in, for example, hair samples. To achieve this a mixture of antibodies and tracers is used. The individual antibodies which form the mixture exhibit cross reactivity towards N-demethyltramadol and O-demethyltramadol, and are derived from immunogens based on N-derivatised tramadol and O-derivatised tramadol, respectively (Table 2). What is surprising about the immunoassay is its high sensitivity compared to commercially available assays, a sensitivity which for tramadol is equal to the sensitivity in reported analytical chemistry based assays and is able to detect each of tramadol, O-demethyltramadol and N-demethyltramadol at concentrations below 5 ng/ml.

**Table 2 Specificity of individual antibodies of the invention towards tramadol and its major metabolites. Antibodies 1 and 2 are raised to immunogens based on O-derivatised tramadol and N-derivatised tramadol, respectively.**

| | **% Cross Reactivity** | | |
|---|---|---|---|
| | Tramadol | O-demethyltramadol | N-demethyltramadol |
| Antibody 1 | 100 | 27 | 2.5 |
| Antibody 2 | 100 | 2.5 | 45.9 |

### DETAILED DESCRIPTION OF THE INVENTION

In a first aspect, the present invention provides immunogens comprising N-demethyltramadol and O-demethyltramadol, each coupled by way of a crosslinker through the nitrogen atom and oxygen atom, respectively, to an antigenicity-conferring material. The invention also provides antibodies raised against such immunogens. In addition, the invention concerns conjugates comprising N-demethyltramadol and O-demethyltramadol, each covalently bonded by way of a crosslinker, to a detectable labelling agent, the crosslinker extending from the nitrogen atom and oxygen atom, respectively.

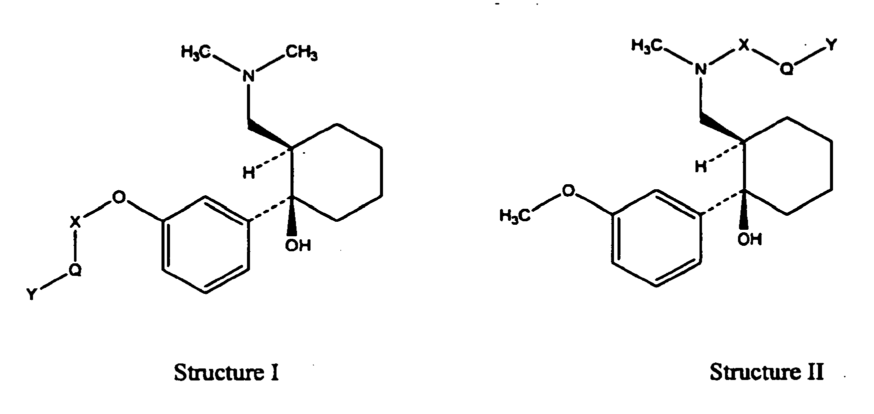

The immunogens and conjugates of the present invention can each be derived from structures of the above formula (the above stereoisomers structures are for representative purposes; the immunogens and conjugates described in the invention are derived from a racemic mixture of *cis*-tramadol) in which the crosslinker (the crosslinker comprises -X-Q) extends from the nitrogen atom of N-demethyltramadol and the oxygen atom of O-demethyltramadol, and couple to the antigenicity-conferring material or the detectable labelling reagent, Y:
in which X is a bivalent link and Q, prior to conjugation with Y, is a reactive group conjugatable with an antigenicity-conferring material (to produce an immunogen) or with a detectable labelling agent (to produce a conjugate).

Preferably, X is a C₁₋₁₀, more preferably a C₂₋₆, most preferably a C₃, substituted or unsubstituted straight chain, saturated alkylene moiety, or an arylene moiety. Advantageously, Q, before conjugation with an antigenicity-conferring material to produce an immunogen, or with a detectable labelling agent to produce a conjugate, is selected from a carboxylic acid, a dithiopyridyl, a maleimide, amino, hydroxyl, thiol, thioester or an aldehyde moiety. Where Q, before conjugation to the antigenicity-conferring material is a carboxylic acid (COOH), the oxygen of the hydroxyl group combines first with DCC and then NHS to form an ester with a powerful leaving group. Nucleophilic attack on the carbonyl group (C=O) of the ester functionality by a free amine group on the antigenicity-conferring material results in an amide bond and formation of the desired immunogen. Conjugate formation follows a similar mechanism using EDC and sulfo-NHS. The skilled reader is referred to Bioconjugate Techniques G. Hermanson, ed., Academic Press, 1996, 785 pp., for details of the interaction between the Q reactive group and either the antigenicity-conferring material or the detectable labelling agent, where Q is selected from the remainder of a carboxylic acid, a dithiopyridyl, a maleimide, amino, hydroxyl, thiol, thioester or an aldehyde moiety. Most advantageously, Q, before conjugation with an antigenicity-conferring material to produce an immunogen, is a carboxy (COOH) moiety. Most advantageously, Q, before conjugation with a detectable labelling agent to produce a conjugate, is a carboxy (COOH) moiety.

The immunogens are prepared by coupling to a modified or non-modified antigenicity-conferring material. Preferably Y, the antigenicity-conferring material contain poly(amino acid) segments and include proteins, protein fragments, glycoproteins, synthetic polypeptides or semi-synthetic polypeptides. Illustrative examples of useful antigenicity-conferring materials are bovine serum albumin (BSA), egg ovalbumin, bovine gamma globulin, bovine thyroglobulin (BTG), keyhole limpet haemocyanin (KLH) etc. Alternatively, synthetic poly(amino acids) having a sufficient number of available amino groups, such as lysine, may be employed, as may other synthetic or natural polymeric materials bearing reactive functional groups. Other representative groups for Y are carbohydrates, yeasts or polysaccharides.

The immunogens obtained are then administered to mammalian hosts to elicit production of specific antibodies, optionally polyclonal antibodies, which are then used to develop immunoassays for tramadol, N-demethyltramadol and O-demethyltramadol, employing labelled conjugates as detection reagents. Preferably, the immunogens are (±)-(*cis*)- 4-{N-[2-hydroxy-2-(3-methoxyphenyl)cyclohexylmethyl]-N-methylamino}butanoic acid and (±)-(*cis*)-4-{3-[1-(N,N-dimethylaminomethyl)-2-hydroxycyclohex-1-yl]phenoxy}butanoic acid coupled to an antigenicity-conferring material, optionally selected from bovine serum albumin (BSA) and bovine thyroglobulin (BTG).

In a still further aspect, the present invention concerns antibodies raised against the immunogens of the present invention, the antibodies being capable of binding with at least one structural epitope of tramadol, N-demethyltramadol and O-demethyltramadol.

In a still further aspect, the present invention concerns antibodies having specificity for tramadol characterised by having cross-reactivity for N-demethyltramadol or O-demethyltramadol. Optionally, the antibodies when combined (and methods and kits including those antibodies) have an equal cross-reactivity for each of N-demethyltramadol and O-demethyltramadol, compared to 100 % cross-reactivity for tramadol.

The invention further provides a process of preparing the antibodies, the process comprising the steps of immunising an animal, preferably a vertebrate animal, most preferably a mammalian animal, by repeated administration of an immunogen of the present invention, and collecting the resulting serum from the immunised animal. Preferably, the process further comprises fixing said serum antibodies to a backing substrate, preferably a solid support, most preferably a polystyrene solid support. Preferably, the antibodies are polyclonal. Alternatively, the antibodies are monoclonal.

In a still further aspect, the present invention comprises conjugates comprising structures I and II in which Y is a detectable labelling agent. Preferably, the labelling agent is selected from an enzyme, a luminescent substance, a radioactive substance, or a mixture thereof. More preferably, the labelling agent is an enzyme, preferably a peroxidase, most preferably horseradish peroxidase (HRP). Alternatively, or additionally, the luminescent substance may be a bioluminescent, chemiluminescent or fluorescent material. The fluorescent substance may be, for example, a monovalent residue of fluorescein or a derivative thereof.

Preferably, the conjugates are (±)-(*cis*)- 4-{N-[2-hydroxy-2-(3-methoxyphenyl)cyclohexylmethyl]-N-methylamino}butanoic acid and (±)-(*cis*)- 4-{3-[1-(N,N-dimethylaminomethyl)-2-hydroxycyclohex-1-yl]phenoxy}butanoic acid covalently bonded to a detectable labelling agent. One such suitable labelling agent is horse-radish peroxidase.

In a still further aspect, the present invention comprises a method for detecting or determining tramadol, N-demethyltramadol and O-demethyltramadol in a sample, the method comprising contacting the sample with at least one conjugate of the present invention, and with at least one antibody of the present invention; detecting or determining bound conjugate; and deducing from a calibration curve the presence of, or the amount of, tramadol, N-demethyltramadol and O-demethyltramadol in the sample. A preferred method for detecting or determining tramadol, N-demethyltramadol and O-demethyltramadol in a sample is by contacting the sample with two conjugates of the present invention with two antibodies of the present invention, said conjugates derived from O-demethyltramadol and N-demethyltramadol, and said antibodies separately raised from an N-demethyltramadol derivatised immunogen of the present invention and an O-demethyltramadol derivatised immunogen of the present invention; and deducing from a calibration curve the presence of, or the amount of, tramadol, N-demethyltramadol and O-demethyltramadol in the sample.

In a further aspect, the invention includes a kit for detecting tramadol, N-demethyltramadol and O-demethyltramadol, the kit including at least one conjugate of the present invention; and at least one antibody of the present invention. The kit may optionally include instructions for the use of said conjugates and said antibodies for detecting an analyte selected from tramadol, N-demethyltramadol, O-demethyltramadol and mixtures of tramadol, N-demethyltramadol and O-demethyltramadol in a sample. Alternatively, the kit is for determining each of tramadol, N-demethyltramadol and O-demethyltramadol, the kit including at least one conjugate of the present invention, and at least one antibody of the present invention. The kit may optionally include instructions for the use of said conjugates and said antibodies for determining the amount of each of tramadol, N-demethyltramadol and O-demethyltramadol in a sample. Preferably the kit for detecting or determining tramadol, N-demethyltramadol and O-demethyltramadol in a sample includes two conjugates of the present invention and two antibodies of present invention, said conjugates derived from N-demethyltramadol and O-demethyltramadol and said antibodies separately raised from an N-demethyltramadol derivatised immunogen of the present invention and an O-demethyltramadol derivatised immunogen of the present invention. The kit may optionally include instructions for the use of said conjugates and antibodies for determining the amount of each of tramadol, N-demethyltramadol and O-demethyltramadol in a sample. More preferably, the sample is serum or urine.

In any individual method and kit of the invention, a compound represented by structures I and II used to prepare the conjugates and the immunogens, against which the antibodies are raised, may be the same or different. In any individual method and kit of the invention, the method or kit can show a sensitivity (represented by the IC₅₀ in ng/ml) of less than 10 ng/ml, optionally less than 5 ng/ml, each for tramadol, N-demethyltramadol and O-demethyltramadol

In a further aspect, the present invention involves use of at least one conjugate according to the present invention, with at least one antibody according to the present invention, to detect or determine tramadol, N-demethyltramadol and O-demethyltramadol in samples such as biological fluids.

### General Procedure for MALDI-TOF Analysis of Immunogens.

In order to confirm that adequate conjugation to an antigenicity-conferring material has been achieved, prior to immunisation, each immunogen is evaluated using matrix-assisted UV laser desorption /ionisation time-of-flight mass spectroscopy (MALDI-TOF MS).
MALDI-TOF mass spectrometry was performed using a Voyager STR Biospectrometry Research Station laser-desorption mass spectrometer coupled with delayed extraction. An aliquot of each sample to be analysed was diluted in 0.1 % aqueous trifluoroacetic acid (TFA) to create 1mg/ml sample solutions. Aliquots (1µl) were analysed using a matrix of sinapinic acid and bovine serum albumin (Fluka) was used as an external calibrant.

### Preparation of Antisera

In order to generate polyclonal antisera, the immunogen of the present invention is mixed with Freund's Adjuvant and the mixture is injected into a host animal, such as rabbit, sheep, mouse, guinea pig or horse. Further injections (boosts) are made and serum is sampled for evaluation of the antibody titre. When the optimal titre has been attained, the host animal is bled to yield a suitable volume of specific antiserum. The degree of antibody purification required depends on the intended application. For many purposes, there is no requirement for purification, however, in other cases, such as where the antibody is to be immobilised on a solid support, purification steps can be taken to remove undesired material and eliminate non-specific binding.

The specific antibodies prepared in this invention are useful as reagents in immunoassays for the detection or determination of tramadol, N-demethyltramadol and O-demethyltramadol in biological fluids. The antibodies of the present invention are capable of binding with the metabolites O-demethyltramadol, N-demethyltramadol and with the parent tramadol.

### DESCRIPTION OF FIGURES

FIGURE 1 shows the preparation of (±)-(*cis*)-4-{3-[1-(N,N-dimethylaminomethyl)-2-hydroxycyclohex-1-yl]phenoxy}butanoic acid
FIGURE 2 shows the preparation of (±)-(*cis*)- 4-{N-[2-hydroxy-2-(3-methoxyphenyl)cyclohexylmethyl]-N-methylamino}butanoic acid
FIGURE 3 shows Immunogen I, (±)-(*cis*)- 4-{N-[2-hydroxy-2-(3-methoxyphenyl)cyclohexylmethyl]-N-methylaminolbutanoic acid conjugated to BTG and Immunogen II, ((±)-(*cis*)-4-{3-[1-(N,N-dimethylaminomethyl)-2-hydroxycyclohex-1-yl]phenoxy}butanoic acid conjugated to BTG
FIGURE 4 shows MALDI results for Immunogen I, (±)-(*cis*)-4-{N-[2-hydroxy-2-(3-methoxyphenyl)cyclohexylmethyl]-N-methylamino}butanoic acid, conjugated to BSA.
FIGURE 5 shows MALDI results for BSA.

### EXAMPLES

### Example 1: Preparation of tramadol free base

Tramadol hydrochloride (5.15 g, 17 mmol) was dissolved in 10% K₂CO₃ solution.

The solution was extracted with EtOAc, dried over Na₂SO₄, and evaporated to dryness to give tramadol (4.43 g, 98%) as a clear oil.

### Example 2: Preparation of O-demethyltramadol

A stock solution of diisobutylaluminium hydride (22 ml, 1.5 M) was added to tramadol (2 g, 76 mmol) in toluene at 0°C. After stirring for 30 mins at 0°C the mixture was warmed to 120°C and refluxed overnight. The reaction mixture was cooled to 0°C and ethanol (20 ml) was slowly added dropwise. After stirring for 10 mins ethanol/water/toluene (1:1:1) was added and the mixture stirred for a further 10 mins. The solids formed were removed by filtration, extracted with EtOAc (2x), washed with water (100 ml), dried over MgSO₄ and evaporated to dryness to give O-demethyltramadol (1.583 g, 84%) as a clear, colourless oil.

### Example 3: Preparation of N-demethyltramadol

To a solution of tramdadol (2.43 g, 9.23 mmol) in toluene (20 ml) was added diethyl azodicarboxylate (2.07 g , 11.89 mmol) and the solution heated at 55°C for 18 h. The mixture was evaporated to dryness, diluted with ethanol (15 ml) and saturated ammonium chloride (15 ml), then heated at reflux for 2 h. The mixture was evaporated to dryness, the residue dissolved in 10% K₂CO₃ solution and water (30 ml), extracted with EtOAc, and drying over Na₂SO₄. On evaporation, a yellow solid was obtained which was purified by liquid chromatography (silica gel; 85:10: 5 EtOAc/MeOH/TEA) to give N-demethyltramadol (1.38 g, 60%) as a yellow solid.

### Example 4: Preparation of (±)-(cis)-Ethyl 4-{3-[1-(N,N-dimethylaminomethyl)-2-hydroxycyclohex-1-yl]phenoxy}butanoate

O-demethyltramadol (0.92 g, 4.13 mmol) was dissolved in acetonitrile (40 ml). Ethyl 4-bromobutanoate (1.21 g, 6.2 mmol) and K₂CO₃ (1.71 g, 12.4 mmol) were added and the mixture heated at reflux overnight.The mixture was filtered, the solvent removed in vacuo and purified by liquid chromatography (silica gel; 10% MeOH in CHCl₃) to give (±)-(*cis*)-Ethyl 3-{3-[1-(N,N-dimethylaminomethyl)-2-hydroxycyclohex-1-yl]phenoxy}propanoate (1.14 g, 86%) as an oil.

### Example 5: Preparation of (±)-(cis)-Ethyl 4-{N-[2-hydroxy-2-(3-methoxyphenyl)cyclohexylmethyl]-N-methylamino}butanoate

N-demethyltramadol (1.38 g, 5.53 mmol), powdered K₂CO₃ (1.68 g, 12.15 mmol) and ethyl 4-bromobutanoate were stirred in DMF (20 ml) at room temperature overnight. The resultant mixture was filtered and EtOAc added to the filtrate. The filtrate was washed with water and brine and the organic layer dried over MgSO₄. After evaporating to dryness, crude mixture was purified by liquid chromatography (silica gel; 5% MeOH in CHCl₃) to give (±)-(*cis*)-Ethyl 4-{N-[2-hydroxy-2-(3-methoxyphenyl)cyclohexylmethyl]-N-methylamino}butanoate (1.23 g, 62%) as an orange oil.

### Example 6: Preparation of (±)-(cis)-4-{3-[1-(N,N-dimethylaminomethyl)-2-hydrozycyclohez-1-yl]phenozy}butanoic acid

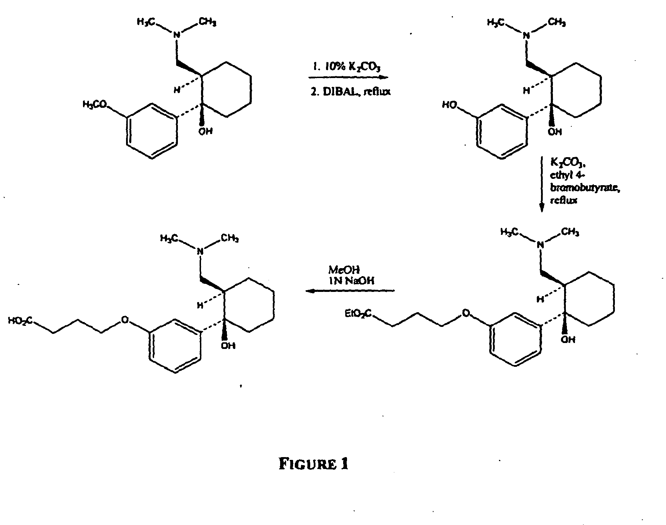

(±)-(*cis*)-Ethyl 4-{3-[1-(N,N-dimethylaminomethyl)-2-hydroxycyclohex-1-yl]phenoxy}butanoate (1.14 g, 3.18 mmol) was dissolved in MeOH (20 ml) and 1N NaOH added. The mixture was stirred at room temperature overnight and solvent removed in vacuo. The remaining aqueous solution was adjusted to pH 3 and extracted with EtOAc. The aqueous solution was neutralised (pH 7), evaporated to dryness, then triturated with 20% MeOH/CHCl₃ and filtered. The filtrate was evaporated to dryness to give (±)-(*cis*)-4-{3-[1-(N,N-dimethylaminomethyl)-2-hydroxycyclohex-1-yl]phenoxy}butanoic acid (0.72 g, 68%) as an off-white solid. See Figure 1.

### Example 7: Preparation of (±)-(cis)- 4-{N-[2-hydroxy-2-(3-methoxyphenyl)cyclohexylmethyl]-N-methylamino}butanoic acid

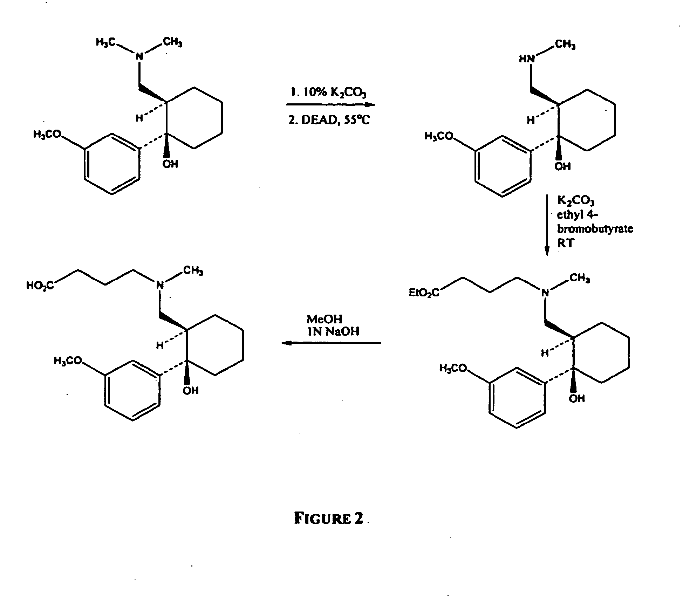

(±)-(*cis*)-Ethyl 4-{N-[2-hydroxy-2-(3-methoxyphenyl)cyclohexylmethyl]-N-methylamino}butanoate (1.23 g, 3.43 mmol) was dissolved in MeOH (20 ml) and 1N NaOH added. The mixture was stirred at room temperature overnight and solvent removed in vacuo. The remaining aqueous solution was adjusted to pH 3 and extracted with EtOAc. The aqueous solution was neutralised (pH 7), evaporated to dryness, 30% MeOH/CHCl₃ added and the mixture stirred for 1 h. After filtration, the filtrate was evaporated to dryness and the residue triturated with ether to give (±)-(*cis*)- 4-{N-[2-hydroxy-2-(3-methoxyphenyl)cyclohexylmethyl]-N-methylamino}butanoic acid (781 mg, 68%) as an off-white solid.
See Figure 2.
**I.R.** v (cm⁻¹) 704, 783, 886, 1194, 1243, 1585, 1608, 2853, 2945, 2964, 3225 ¹³C NMR (CDCl₃) δ (ppm) 174.5, 158.9, 151.3, 128.6, 117.2, 111, 110.8, 74.4, 58.4, 57.1, 54.8, 42.8, 41.0, 39.7, 32.2, 26.3, 25.5, 21.9, 21.7

### Example 8: Conjugation of (±)-(cis)- 4-{N-[2-hydroxy-2-(3-methoxyphenyl)cyclohexylmethyl]-N-methylamino}butanoic acid to BSA

To a solution of (±)-(*cis*)- 4-{N-[2-hydroxy-2-(3-methoxyphenyl)cyclohexylmethyl]-N-methylamino}butanoic acid (45.39 mg, 0.135 mol) in DMF (3 ml) was added N-hydroxy succinimide (17.03 mg, 0.148 mol) and N,N-dicyclohexylcarbodiimide (DCC) (30.53 mg, 0.148 mol) and the mixture stirred at room temperature overnight.

The dicyclohexylurea formed was removed by filtration and the filtrate added dropwise to a solution of BSA (150 mg, 2.28 mmol) in 0.1M sodium bicarbonate (pH 8.5, 9 ml). The mixture was stirred at room temperature overnight. The solution was dialysed against 50 mM phosphate buffer, pH 7.2 (3 changes) for 24 hours at 4°C and stored at -40°C.

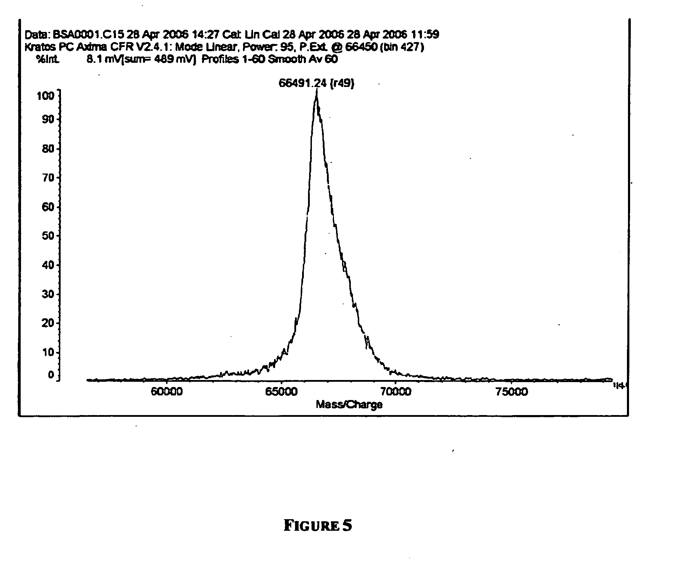

**MALDI:** results showed that 8.1 molecules of (±)-(*cis*)- 4-{N-[2-hydroxy-2-(3-methoxyphenyl)cyclohexylmethyl]-N-methylamino}butanoic acid were conjugated to 1 molecule of BSA. Specifically, a major signal was present which indicates a protonated mass for this sample of m/z 69,063. See Figure 5.

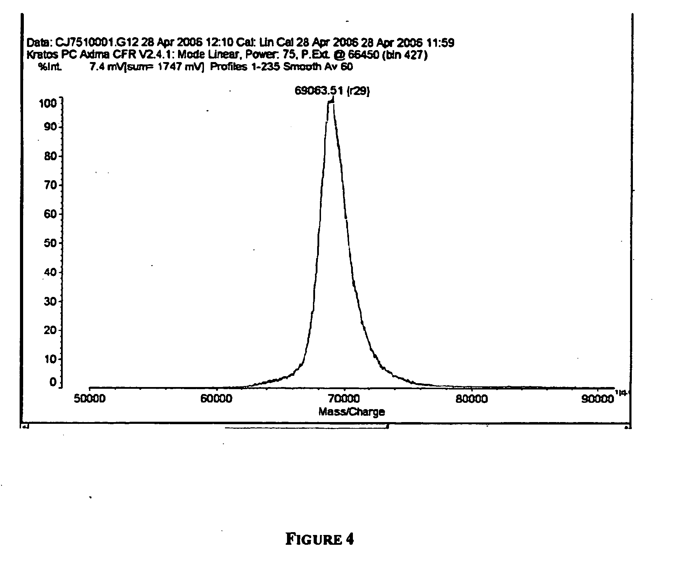

### Example 9: Conjugation of (±)-(cis)- 4-{N-[2-hydroxy-2-(3-methoxyphenyl)cyclohexylmethyl]-N-methylamino}butanoic acid to BTG (Immunogen I - Figure 4)

The conjugation of (±)-(*cis*)- 4-{N-[2-hydroxy-2-(3-methoxyphenyl)cyclohexylmethyl]-N-methylamino}butanoic acid to BTG was carried out by the same method as Example 7 by using: (±)-(*cis*)- 4-{N-[2-hydroxy-2-(3-methoxyphenyl)cyclohexylmethyl]-N-methylamino}butanoic acid (52.66 mg), N-hydroxysuccinimide (19.79 mg), DCC (35.48 mg) and BTG (150 mg).

### Example 10: Conjugation of (±)-(cis)-4-{3-[1-(N,N-dimethylaminomethyl)-2-hydroxycyclohex-1-yl]phenoxy}butanoic acid to BSA

To a solution of (±)-(*cis*)-4-{3-[1-(N,N-dimethylaminomethyl)-2-hydroxycyclohex-1-yl]phenoxy}butanoic acid (45.28 mg, 0.135 mol) in DMF (1 ml) was added N-hydroxy succinimide (17.03 mg, 0.148 mol) and N,N-dicyclohexylcarbodiimide (DCC) (30.53 mg, 0.148 mol) and the mixture stirred at room temperature overnight. The dicyclohexylurea formed was removed by filtration and the filtrate added dropwise to a solution of BSA (150 mg, 2.28 mmol) in 0.1M sodium bicarbonate (pH 8.5, 9 ml). The mixture was stirred at room temperature overnight. The solution was dialysed against 50 mM phosphate buffer, pH 7.2 (3 changes) for 24 hours at 4°C and stored at -40°C.
**MALDI:** results showed that 23.3 molecules of (±)-(*cis*)-4-{3-[1-(N,N-dimethylaminomethyl)-2-hydroxycyclohex-1-yl]phenoxy}butanoic acid were conjugated to 1 molecule of BSA. Specifically, a major signal was present which indicates a protonated mass for this sample of m/z 73,690.

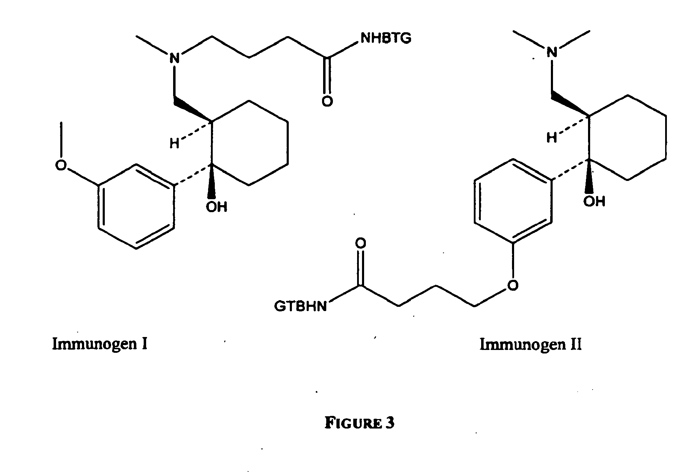

### Example 11: Conjugation of (±)-(cis)-4-{3-[1-(N,N-dimethylaminomethyl)-2-hydroxycyclohex-1-yl]phenoxy}butanoic acid to BTG (Immunogen II - Figure 3)

The conjugation of (±)-(*cis*)-4-{3-[1-(N,N-dimethylaminomethyl)-2-hydroxycyclohex-1-yl]phenoxy}butanoic acid (±)-(*cis*)-4-{3-[1-(N,N-dimethylaminomethyl)-2-hydroxycyclohex-1-yl]phenoxy}butanoic acid to BTG was carried out by the same method as Example 7 by using: (±)-(*cis*)-4-{3-[1-(N,N-dimethylaminomethyl)-2-hydroxycyclohex-1-yl]phenoxy}butanoic acid (52.66 mg), N-hydroxysuccinimide (19.79 mg), DCC (35.48 mg) and BTG (150 mg).

### Example 12: Conjugation of {N-[2-hydroxy-2-(3-methoxyphenyl)cyaohexylmethyl]-N-methylamino}butanoic acid to HRP (Conjugate I)

EDC.HCl (10 mg) was dissolved in water (0.6 ml) and immediately added to a solution of {N-[2-hydroxy-2-(3-methoxyphenyl)cyclohexylmethyl]-N-methylamino}butanoic acid (2 mg) in DMF (0.2 ml). After mixing this solution was added dropwise to a solution of HRP (20 mg) in water (1 ml). Sulfo-NHS (5 mg) was added and the reaction mixture was incubated in the dark at room temperature overnight. Excess {N-[2-hydroxy-2-(3-methoxyphenyl)cyclohexylmethyl]-N-methylamino}butanoic acid was removed by desalting with PD-10 columns (Pharmacia) in series, pre-equilibrated with PBS (phosphate buffered saline) at pH 7.2. The {N-[2-hydroxy-2-(3-methoxyphenyl)cyclohexylmethyl]-N-methylamino}butanoic acid -HRP conjugate was then dialysed overnight against 10 L of PBS/ pH 7.2 at 4°C.

### Example 13: Conjugation of (±)-(cis)-4-{3-[1-(N,N-dimethylaminomethyl)-2-hydroxycydohex-1-yl]phenoxy}butanoic acid to HRP (Conjugate II)

A solution of 1 M potassium hydroxide (100 ml) was added to a solution to p-(3-acetylthiopropanamido)leucomalachite (2 mg) in DMF (0.2 ml). After incubating for 10 minutes, 0.1 M phosphate buffer was added, followed by 1 M HCl. After mixing, this solution was added dropwise to a solution of modified HRP (20 mg). Excess hapten was removed by desalting with PD-10 columns (Pharmacia) in series, pre-equilibrated with PBS (phosphate buffered saline) at pH 7.2. The hapten-HRP conjugate was then dialysed overnight against 10 L of PBS/ pH 7.2 at 4°C.

### Example 14: Preparation of antibodies to Immunogens I and II, prepared in Examples 9 and 11

An aqueous solution of Immunogen **I** was formulated with Freund's Complete Adjuvant (FCA) to form an emulsion consisting of 2 mg/ml immunogen in 50% (v/v) FCA. Three sheep were immunised with this emulsion (1° immunisation), 0.25 ml being intramuscularly injected at each of four sites in the rump of each animal. Subsequent immunizations (boosts) contained 1mg/ml immunogen. All boosts were emulsified in 50% (v/v) Freund's Incomplete Adjuvant (FIA) and were administered in the same manner as the 1° immunisation, at monthly intervals for 1 year. Blood sampling took place 7 to 14 days after each boost. Each sample was processed to produce antiserum, which was further purified by caprylic acid and ammonium sulfate precipitation to yield an immunoglobulin (Ig) fraction. The Ig fraction was evaluated by competitive ELISA microtiter plate assay, as described in Example 15 below. Immunogen **II** was subject to the same protocol.

### Example 15: Development of ELISAs for Tramadol, N-demethyltramadol and O-demethyltramadol

The wells of an enhanced binding 96 well polystyrene microtiter plate were coated with a mixture of the Ig fractions of the antisera raised to immunogens I and II, diluted in 10 mM Tris, pH8.5 (125 µl/well). The appropriate antibody coating dilution was determined using standard ELISA checkerboard techniques. The plate was incubated for 2 hours at 37°C, washed 4 times with Tris buffered saline containing Tween 20 (TBST) and tapped dry. Standard solutions of O-demethyltramadol and N-demethyltramadol were prepared in TBST at 0, 0.1, 1.0, 5, 10, 25, 50 and 100 ng/ml, and 50 µl of each was added to the appropriate wells.

A mixture of conjugates I and II, diluted in Tris buffer (pH 7.2) containing EDTA, D-mannitol, sucrose, thimerosal and BSA, was added to each of the wells. The appropriate dilution of conjugate was also determined using standard ELISA checkerboard techniques. The plate was incubated at 25°C for 1 hour. Excess unbound conjugate was removed by washing 6 times over a 10 minute period with TBST. 125 µl of tetramethylbenzidine (TMB) substrate solution was added to each well of the plate that was then incubated for 20 minutes in the dark at room temperature. The reaction was terminated by addition of 125 µl 0.2M H₂SO₄ to each well. The absorbance was then measured at 450 nm using a microtiter plate reader.

**Table 3 Sensitivity of commercial assays towards tramadol and metabolites compared to inventors' assay.**

| | **Sensitivity ng/ml** | | | |
|---|---|---|---|---|
| | Tramadol | O-Demethyltramadol | N-Demethyltramadol | Metabolite ratio |
| IDS* | 200 | 500 | >10000 | >1 : 20 |
| Bio-Quant⁺ | 577 | 75 | >1500 | >1 : 20 |
| Neogen* | 5 | 18.5 | 83.3 | 1 : 4.5 |
| Randox* | 0.3 | 3 | 3.4 | 1:1.1 |

| | | | | |
|---|---|---|---|---|
| Sensitivity calculated from cross-reactivity data * Sensitivity is equal to IC₅₀ ⁺ Sensitivity is equal to the absorbance of the standard alone (A₀) plus 4 standard deviations | | | | |

### BIBLIOGRAPHY

1. Senay E.C., Adams E.H., Geller A., Inciardi J.A., MunozA., Schnoll S.H. and Cicero T.J.(2003) Physical dependence on Ultram (tramadol hydrochloride): both opioid-like and atypical withdrawal symptoms occur. Drug and Alcohol Dependency 69(3): 233-41
2. Freye E. and Levy J. (2000). Acute abstinence syndrome following abrupt cessation of long-term use of tramadol (Ultram): a case study. European Journal of Pain, 4(3): 307-311.
3. Subrahmanyam V., Renwick A.B., Walters D.G., Young P.J., Price R.J., Tonelli A.P. and Lake B.G. (2001). Identification of cytochrome P-450 isoforms responsible for cis-tramadol metabolism in human liver microsomes. Drug Metabolism and Disposition, 29(8): 1146-1155.
4. Enggaard T.P., Poulsen L., Arendt-Nielsen L., Brosen K., Ossig J. and Sidrup S.H. (2006). The analgesic effect of tramadol after intravenous injection in healthy volunteers in relation to CYP2D6. Anesthesia and Analgesia, 102(1): 146-150.
5. Tao Q., Stone D.J., Borenstein M.R., Jean-Bart V., Codd E.E., Coogan T.P., Desai-Krieger D., Liao S. and Raffa R.B. Gas chromatographic method using nitrogen-phosphorous detection for the measurement of tramadol and its O-demethyl metabolite in plasma and brain tissue of mice and rats. Journal of Chromatography B Biomedical Science Applications, 763(1-2): 165-171.
6. Leis H.J., Fauler G. and Windischhofer W. (2004). Synthesis of d1-N-ethyltramadol as an internal standard for the quantitative determination of tramadol in human plasma by gas chromatography-mass spectrometry. Journal of Chromatography B Analytical Technology and Biomedical Life Sciences, 804(2): 369-74.
7. Zhao L.M., Chen X.Y., Cui J.J., Sunita M. and Zhong D.F. (2004). Determination of tramadol and its active metabolite in plasma and amniotic fluid using LC/MS/MS. Yao Xue Xue Bao, 39(6): 458-62.
8. Gu Y. and Fawcett J.P. (2005). Improved HPLC method for the simultaneous determination of tramadol and O-demethyltramadol in human plasma. Journal of Chromatography B Analytical Technology and Biomedical Life Sciences, 821(2): 240-243.
9. Sha Y.F., Shen S. and Duan G.L. (2005). Rapid determination of tramadol in human plasma by headspace solid-phase microextraction and capillary gas chromatography-mass spectrometry. Journal of Pharmaceutical and Biomedical Analysis, 37(1): 143-147.
10. Hadidi K.A., Almasad J.K., Al-Nousr T. and Abu-Ragheib S (2000). Determination of tramadolin hair using solid phase extraction and GC-MS. Forensic Science International, 135(2): 129-136.
11. Eysseric H., Kintz P., Goulle J.P., Vincent F. and Barret L. (2001). Tramadol Concentrations, Including Hair Analysis, in Six Fatal and Non-Fatal Intoxications. 39th Annual TIAFT Meeting, Prague.
12. Moore C., Rana S. and Coulter C. (2007). Determination of meperidine, tramadol and oxycodone in human oral fluid using solid-phase extraction and gas chromatography-mass spectrometry. Journal of Chromatography B, 850: 370-375.

## Claims

1. A method for detecting or determining tramadol, N-demethyltramadol and O-demethyltramadol in a sample, the method comprising contacting the sample with two conjugates of structures I and II and with two antibodies raised from immunogens of structures I and II; detecting bound conjugates; and deducing from a calibration curve the presence of, or the amount of, tramadol, N-demethyltramadol and O-demethyltramadol in the sample, 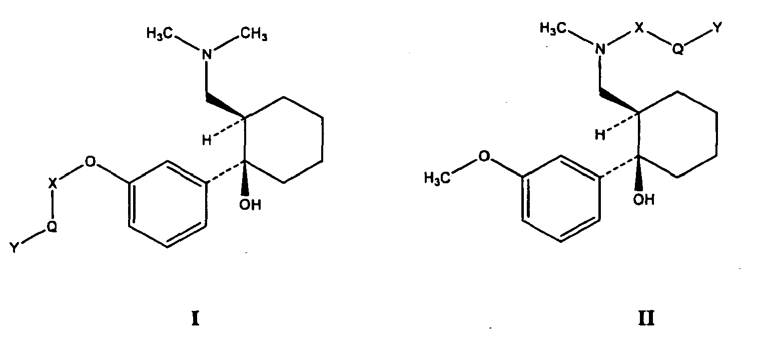 wherein,
X is a C₁₋₁₀, more preferably a C₂₋₆, most preferably a C₃, substituted or unsubstituted straight chain, saturated alkylene moiety, or an arylene moiety,
Q, before connection to either a detectable labelling agent or an antigenicity conferring material, is selected from a carboxy, a dithiopyridyl, a maleimide, amino, hydroxyl, thiol, thioester or an aldehyde moiety and,
Y is a detectable labelling agent forming a conjugate, or an antigenicity-conferring material forming an immunogen.

2. The method of Claim 1 in which the conjugates are (±)-(*cis*)- 4-{N-[2-hydroxy-2-(3-methoxyphenyl)cyclohexylmethyl]-N-methylamino}butanoic acid connected to a detectable labelling agent, and (±)-(*cis*)-4-{3-[1-(N,N-dimethylaminomethyl)-2-hydroxycyclohex-1-yl]phenoxy}butanoic acid connected to a detectable labelling agent.

3. The method of Claim 1 or 2 in which the detectable labelling agent is selected from an enzyme, which is preferably a peroxidase, most preferably horseradish peroxidase; a luminescent substance; or a radioactive substance.

4. The method of Claim 1 in which one immunogen is (±)*-*(*cis*)- 4-{N-[2-hydroxy-2-(3-methoxyphenyl)cyclohexylmethyl]-N-methylamino}butanoic acid coupled to an antigenicity-conferring material and the other is (±)-(*cis*)-4-{3-[1-(N,N-dimethylaminomethyl)-2-hydroxycyclohex-1-yl]phenoxy}butanoic acid coupled to an antigenicity-conferring material.

5. The method of Claim 1 or 4 in which the antigenicity-conferring material is selected from a protein, a protein fragment, a synthetic polypeptide or a semisynthetic polypeptide.

6. The method of Claim 1 in which one antibody is raised against immunogen 1, the antibody having specificity for tramadol **characterised by** having cross-reactivity for O-demethyltramadol, and the other antibody is raised against immunogen II, the antibody having specificity for tramadol **characterised by** having cross-reactivity for N-demethyltramadol.

7. The method of Claim 1 in which one antibody is raised against (±)-(*cis*)- *4-*{N-[2-hydroxy-2-(3-methoxyphenyl)cyclohexylmethyl]-N-methylamino}butanoic acid coupled to an antigenicity-conferring material, the antibody having specificity for tramadol **characterised by** having cross-reactivity for N-demethyltramadol, and the other antibody is raised against (±)-(*cis*)-4-{3-[1-(N,N-dimethylaminomethyl)-2-hydroxycyclohex-1-yl]phenoxy}butanoic acid coupled to an antigenicity-conferring material, the antibody having specificity for tramadol **characterised by** having cross-reactivity for O-demethyltramadol.

8. The method of Claim I comprising detecting or determining tramadol, N-demethyltramadol and O-demethyltramadol in a sample, the method comprising contacting the sample with the conjugates (±)-(*cis*)-4-{3-[1-(N, N-dimethylaminomethyl)-2-hydroxycyclohex-1-yl]phenoxy}butanoic acid and (±)-(*cis*)-4-{N-[2-hydroxy-2-(3-methoxyphenyl)cyclohexylmethyl]-N-methylamino}butanoic acid each connected to detectable labelling agent, and a mixture of antibodies raised from the immunogens (±)-(*cis*)-4-{3-[1-(N,N-dimethylaminomethyl)-2-hydroxycyclohex-1-yl]phenoxy}butanoic acid and (±)-(*cis*)- 4-{N-[2-hydroxy-2-(3-methoxyphenyl)cyclohexylmethyl]-N-methylamino}butanoic acid each connected to an antigenicity-conferring material; detecting bound conjugates; and deducing from a calibration curve the presence of, or the amount of, tramadol, N-demethyltramadol and O-demethyltramadol in the sample.

9. A kit for detecting or determining tramadol, N-demethyltramadol and O-demethyltramadol in a sample, the kit **characterised by** including two conjugates of claim 1 or 2 and two antibodies of claim 6 or 7.

## Patentansprüche

1. Ein Verfahren zur Messung und Bestimmung von Tramadol, N-Desmethyltramadol und O-desmethyltramadol in einer Probe, wobei das Verfahren den Kontakt der Probe mit zwei konjugierten Strukturen I und II und mit zwei Antikörpern, die aus den Immunogenen aus den Strukturen I und II hergestellt werden, und die Messung der gebundenen Konjugate und die Ableitung der Anwesenheit bzw. der Menge an Tramadol, N-Desmethyltramadol und O-desmethyltramadol in der Probe anhand einer Kalibrierungskurve umfasst. 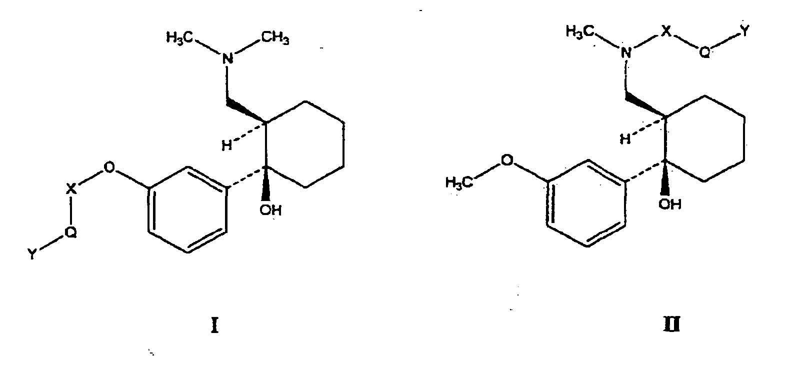 wobei:
X ein gesättigter Alkylenrest oder ein Arylenrest mit einer durch C₁₋₁₀, besser C₂₋₆ oder noch besser C₃ substituierten oder nichtsubstituierten geraden Kette ist,
Q vor der Verbindung mit entweder einem messbaren Kennzeichnungsstoff oder einem Antigenität verleihenden Material aus einem Karboxy-, Dithiopyridil-, Maleimid-, Amino-, Hydroxyl-, Thiol-, Thioester- oder einem Aldehydrest ausgewählt wird und
Y ein messbarer Kermzeichnungsstoff, der ein Konjugat bildet, oder ein Antigenität verleihendes Material ist, das ein Immunogen bildet.

2. Das Verfahren aus Anspruch 1, bei dem die Konjugate sind (+/-)-(cis)-4-{N-[2-Hydroxy-2-(3-Methoxyphenyl)cyclohexylmethyl]-N-Methylamino}Butansäure, verbunden mit einem messbaren Kennzeichnungsstoff, und (+/-)-(cis)-4-{3-[1-(N,N-Dimethylaminomethyl)-2-Hydroxycyclohex-1-yl]phenoxy}Butansäure sind, verbunden mit einem messbaren Kennzeichnungsstoff.

3. Das Verfahren aus Anspruch 1 oder 2, in dessen Rahmen ein messbarer Kennzeichnungsstoff aus einem Enzym ausgewählt wird, wobei es sich vorzugsweise um Peroxidase, am besten Meerrettich-Peroxidase; eine leuchtende Substanz; oder eine radioaktive Substanz handelt.

4. Das Verfahren aus Anspruch 1, in dessen Rahmen ein Immunogen (+/-)-(cis)-4-{N-[2-Hydroxy-2-(3-Methoxyphenyl)cyclohexylmethyl]-N-Methylamino}Butansäure ist, gekoppelt an ein Antigenität verleihendes Material, und das andere Immunogen (+/-)-(cis)-4-{3-[1-(N,N-Dimethylaminomethyl)-2-Hydroxycyclohex-1-yl]phenoxy}Butansäure ist, gekoppelt an ein Antigenität verleihendes Material.

5. Das Verfahren aus den Ansprüchen 1 oder 4, in dessen Rahmen ein Antigenität verleihendes Material aus einem Protein, einem Proteinfragment, einem künstlich hergestellten Polypeptid oder einen halb-künstlich hergestellten Polypeptid ausgewählt wird.

6. Das Verfahren aus Anspruch 1, in dessen Rahmen ein Antikörper gegen Immunogen 1 ausgelöst wird, wobei der Antikörper eine spezifische Wirksamkeit für Tramadol aufweist und **dadurch gekennzeichnet ist, dass** er ein Reaktionsvermögen über Kreuz für 0-Desmethyltramadol besitzt, und der andere Antikörper gegen Immunogen II ausgelöst wird, wobei der Antikörper eine spezifische Wirksamkeit für Tramadol aufweist und
**dadurch gekennzeichnet ist, dass** er ein Reaktionsvermögen über Kreuz für N-Desmethyltramadol besitzt.

7. Das Verfahren aus Anspruch 1, in dessen Rahmen ein Antikörper gegen (+/-)-(cis)-4-{N-[2-Hydroxy-2-(3-Methoxvphenyl)cyclohexylmethyl]-N-Methylamino}Butansäure ausgelöst wird, gekoppelt an ein Antigenität verleihendes Material, wobei der Antikörper eine spezifische Wirksamkeit für Tramadol aufweist und **dadurch gekennzeichnet ist, dass** er ein Reaktionsvermögen über Kreuz für N-Desmethyltramadol besitzt, und der andere Antikörper gegen (+/-)-(cis)-4-{3-[1-(N,N-Dimethylaminomethyl)-2-Hydroxycyclohex-1-yl]phenoxy}Butansäure ausgelöst wird, gekoppelt an ein Antigenität verleihendes Material, wobei der Antikörper eine spezifische Wirksamkeit für Tramadol aufweist und **dadurch gekennzeichnet ist, dass** er ein Reaktionsvermögen über Kreuz für 0-Desmethyltramadol besitzt

8. Das Verfahren aus Anspruch 1, das die Messung und Bestimmung von Tramadol, N-Desmethyltramadol und 0-Desmethyltramadol in einer Probe umfasst, wobei das Verfahren Folgendes umfasst: die Probe mit den Konjugaten (+/-)-(cis)-4-{3-[1-(N,N-Dimethylaminomethyl)-2-Hydroxycyclohex-1-yl]phenoxy}Butansäure und (+/-)-(cis)-4-{N-[2-Hydroxy-2-(3-Methoxyphenyl)cyclohexylmethyl]-N-Methylamino}Butansäure, beide verbunden mit einem messbaren Kennzeichnungsstoff, und einer Mischung aus Antikörpern in Kontakt bringen, die aus den Immunogenen (+/-)-(cis)-4-{3-[1-(N,N-Dimethylaminomethyl)-2-Hydroxycyclohex-1-yl]phenoxy}Butansäure und (+/-)-(cis)-4-{N-[2-Hydroxy-2-(3-Methoxyphenyl)cyclohexylmethyl]-N-Methylamino}Butansäure, beide verbunden mit einem Antigenität verleihenden Material hergestellt wurden, die Messung der gebundenen Konjugate, und die Ableitung der Anwesenheit bzw. der Menge an Tramadol, N-Desmethyltramadol und 0-Desmethyltramadol in der Probe anhand einer Kalibrierungskurve.

9. Ein Satz zur Messung und Bestimmung von Tramadol, N-Desmethyltramadol und 0-Desmethyltramadol in einer Probe, wobei der Satz **dadurch gekennzeichnet ist, dass** er zwei Konjugate aus den Ansprüchen 1 und 2 und zwei Antikörper aus den Ansprüchen 6 und 7 enthält.

## Revendications

1. Méthode de détection ou de détermination de tramadol, de N-demethyltramadol et d'O-demethyltramadol dans un échantillon, la méthode comprenant le contact de l'échantillon avec deux conjugués de structures 1 et II et avec deux anticorps dirigés contre les immunogènes des structures I et II ; la détection de conjugués attachés ; et la déduction à partir d'une courbe d'étalonnage de la présence de ou de la quantité de tramadol, de N-demethyltramadol et d'O-demethyltramadol dans l'échantillon, 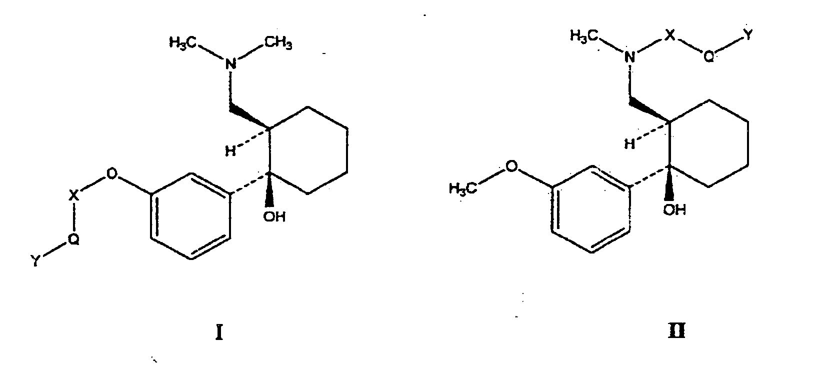 où,
X est un C₁₋₁₀, et plus de préférence un C₂₋₆, et au mieux un C₃, à chaîne linéaire substituée ou non substituée, un motif alkylène saturé ou un motif arylène,
Q, avant la conjugaison à un agent de marquage détectable ou à une substance conférant l'antigénicité, est choisi parmi un motif carboxy, dithiopyridyle, maléimide, amino, hydroxyle, thiol, thioester ou aldéhyde et,
Y est un agent de marquage détectable créant un conjugué ou un matériel conférant de l'antigénicité créant un immunogène.

2. Méthode selon la Revendication 1, dans laquelle les conjugués sont de l'acide (±)-(cis)-4-{N-[2-hydroxy-2-(3-méthoxyphényl)cyclohéxylméthyl]-N-méthylamino}butanoïque couplé à un agent de marquage détectable et de l'acide (±)-(cis)-4-{3-[1-(N,N-diméthylaminométhyl)-2-hydroxycyclohex-1-yl]phénoxy}butanoïque couplé à un agent de marquage détectable.

3. Méthode selon la Revendication 1 ou 2 dans laquelle l'agent de marquage détectable est choisi parmi une enzyme qui est de préférence une peroxydase, plus de préférence la peroxydase de raifort : une substance luminescente ; ou une substance radioactive.

4. Méthode selon la Revendication 1 dans laquelle un immunogène est l'acide (±)-(cis)-4-{N-[2-hydroxy-2-(3-méthoxyphényl)cyclohéxylméthyl]-N-méthylamino}butanoïque couplé à un matériel conférant de l'antigénicité et l'autre est de l'acide (±)-(cis)-4-{3-[1-(N,N-diméthylaminométhyl)-2-hydroxycyclohex-1-yl]phénoxy}butanoïque couplé à un matériel conférant de l'antigénicité.

5. Méthode selon la Revendication 1 ou 4 dans laquelle le matériel conférant de l'antigénicité est choisi parmi une protéine, un fragment protéique, un polypeptide synthétique ou un polypeptide semi-synthétique.

6. Méthode selon la Revendication 1 dans laquelle un anticorps est dirigé contre l'immunogène I, l'anticorps ayant spécificité pour tramadol **caractérisé par** la réactivité croisée pour O-deméthyltramadol et l'autre anticorps est dirigé contre immunogène II, l'anticorps ayant spécificité pour tramadol **caractérisé par** la réactivité croisée pour N-deméthyltramadol.

7. Méthode selon la Revendication 1 dans laquelle un anticorps est dirigé contre l'acide (±)-(cis)-4-{N-[2-hydroxy-2-(3-méthoxyphényl)cyclohéxylméthyl]-N-méthylamino}butanoïque couplé à un matériel conférant de l'antigénicité, l'anticorps ayant spécificité pour tramadol **caractérisé par** la réactivité croisée pour N-démethyltramadol et l'autre anticorps est dirigé contre l'acide (±)-(cis)-4-{3-[1-(N,N-diméthylaminométhyl)-2-hydroxycyclohex-1-yl]phénoxy}butanoïque couplé à un matériel conférant de l'antigénicité, l'anticorps ayant pécificité pour tramadol **caractérisé par** la réactivité croisée pour O-deméthyltramadol.

8. Méthode selon la Revendication 1 comprenant la détection ou la détermination de tramadol, N-demethyltramadol et O-demetyhltramadol dans un échantillon, la méthode comprenant le contact de l'échantillon avec les conjugués l'acide (±)-(cis)-4-{3-[1-(N,N-diméthylaminométhyl)-2-hydroxycyclohex-1-yl]phénoxy}butanoïque et l'acide (±)-(cis)-4-{N-[2-hydroxide-2-(3-méthoxyphényl)cyclohéxylméthyl]-N-méthylamino}butanoïque, chacun couplé à un agent de marquage détectable, et un mélange d'anticorps dirigé contre les immunogènes l'acide (±)-(cis)-4-{3-[1-(N,N-diméthylaminométhyl)-2-hydroxycyclohex-1-yl]phénoxy}butanoïque et l'acide (±)-(cis)-4-{N-[2-hydroxide-2-(3-méthoxyphényle)cyclohéxyleméthyle]-N-méthylamino}butanoïque, chacun connecté à un matériel conférant de l'antigénicité ; la détection de conjugués attachés ; et la déduction à partir d'une courbe d'étalonnage de la présence de ou de la quantité de tramadol, de N-demethyltramadol et d'O-demethyltramadol dans l'échantillon.

9. Un kit pour détecter ou déterminer le tramadol, le N-demethyltramadol et l'O-demethyltramadol dans un échantillon, le kit étant **caractérisé par** l'inclusion de deux conjugués de la revendication 1 ou 2 et deux anticorps de la revendication 6 ou 7.
